# EUROPEAN PATENT APPLICATION

(11) **EP 4 822 995 A1**
(43) Date of publication of application: **14.10.2026**
(21) Application number: 25741598.4
(22) Date of filing: 17.01.2025
(51) Int. Cl.: H03M 3/00, H03H 7/06, H03F 3/68, A61N 1/05, A61N 1/36

(54) **SIGNAL COLLECTION CIRCUIT, CONTACT COMBINATION METHOD, STIMULATOR, AND IMPLANTABLE MEDICAL SYSTEM AND DEVICE**

(30) Priority: 19.01.2024 CN 202410077315
(71) Applicant: Sceneray Co., Ltd, Suzhou, Jiangsu 215000 (CN)
(72) Inventor: ZHI, Menghui, Suzhou, Jiangsu 215000 (CN); ZHU, Weiran, Suzhou, Jiangsu 215000 (CN)
(74) Representative: Cabinet Beaumont
(86) International application number: PCT/CN2025/072904
(87) International publication number: WO 2025/153037

(57) **Abstract**

Provided are a signal acquisition circuit, a contact combination method, a stimulator, and an implantable medical system and device. The signal acquisition circuit includes a multi-contact electrode, an analog switch, a sampling capacitor array, a multiplexer, and a signal sampling module. At least part of contacts of the multi-contact electrode are inserted into a target nucleus. All the contacts of the multi-contact electrode are electrically connected to all sampling capacitors in the sampling capacitor array in a one-to-one correspondence through the analog switch. Each sampling capacitor in the sampling capacitor array is electrically connected to the multiplexer, so as to enable the multiplexer to select any two sampling capacitors. The multiplexer is electrically connected to the signal sampling module.

## Description

This application claims priority to Chinese Patent Application No. 202410077315.X filed with the China National Intellectual Property Administration (CNIPA) on Jan. 19, 2024, the disclosure of which is incorporated herein by reference in its entirety.

### TECHNICAL FIELD

The present application relates to the technical field of medical devices, for example, a signal acquisition circuit, a contact combination method, a stimulator, and an implantable medical system and device.

### BACKGROUND

Stimulators are a type of implantable device. A stimulator, which includes an implantable pulse generator (IPG) and an electrode lead, can provide a patient with a refined electrical stimulation therapy with controllable parameters and is recognized by many patients suffering from Parkinson's disease in the market.

To implement real-time closed-loop stimulation, contacts on an electrode are required not only to perform electrical stimulation but also to acquire local field potential (LFP) signals from the subthalamic nucleus (STN). For a multi-contact electrode with N contacts (N ≥ 2), differences among patients and variations in surgery performed by surgeons introduce uncertainties as to the position at which the electrode is inserted into the STN, and it is impossible to determine which two contacts (configured in a differential mode to reduce common-mode noise) provide an optimal LFP acquisition position. Therefore, before closed-loop control is performed, multiple contact combinations need to be cyclically sampled, and an optimal acquisition contact combination is determined comprehensively based on an LFP signal amplitude and a signal-to-noise ratio. To cyclically sample multiple contact combinations, two methods are currently available. In method one, a signal processing circuit is provided at the back end of each electrode contact combination. In method two, two multiplexers are used to select any two electrode contacts and a processing circuit is shared at the back end. Method one can ensure that all electrode contact combinations are sampled at the same moment, thereby implementing an accurate determination process of the optimal acquisition contact combination. However, N*(N - 1)/2 signal processing circuits are clearly inconsistent with the requirements of IPG design for small volume, light weight, and low power consumption. Method two can reduce the scale of IPG circuits. However, LFP data analyzed for each acquisition contact combination are actually acquired at different moments (because the processing circuit is shared and cyclical sampling and analysis are required). It is difficult to ensure that a determination process of the optimal acquisition contact combination is completely accurate.

### SUMMARY

The present application provides a signal acquisition circuit, a contact combination method, a stimulator, and an implantable medical system and device.

The present application provides a signal acquisition circuit. The signal acquisition circuit includes a multi-contact electrode, an analog switch, a sampling capacitor array, a multiplexer, and a signal sampling module.

At least part of contacts of the multi-contact electrode are inserted into a target nucleus.

All the contacts of the multi-contact electrode are electrically connected to all sampling capacitors in the sampling capacitor array in a one-to-one correspondence through the analog switch.

Each sampling capacitor in the sampling capacitor array is electrically connected to the multiplexer, so as to enable the multiplexer to select any two sampling capacitors.

The multiplexer is electrically connected to the signal sampling module.

In one or more embodiments, the multiplexer includes a first multiplexer and a second multiplexer.

Each sampling capacitor in the sampling capacitor array is electrically connected to the first multiplexer and the second multiplexer separately, and an output terminal of the first multiplexer and an output terminal of the second multiplexer are each connected to the signal sampling module.

In one or more embodiments, the signal sampling module includes a first voltage follower, a second voltage follower, a pre-filter, a differential amplifier, a low-pass filter, a programmable amplifier, and an analog-to-digital converter.

The first multiplexer is electrically connected to the first voltage follower.

The second multiplexer is electrically connected to the second voltage follower.

The first voltage follower and the second voltage follower are electrically connected to the pre-filter separately.

The pre-filter is electrically connected to the differential amplifier.

The differential amplifier is electrically connected to the low-pass filter.

The low-pass filter is electrically connected to the programmable amplifier.

The programmable amplifier is electrically connected to the analog-to-digital converter.

In one or more embodiments, the multi-contact electrode includes N contacts, the analog switch includes N channels, the sampling capacitor array includes N sampling capacitors, and each of the first multiplexer and the second multiplexer is an N-to-1 multiplexer.

One terminal of the M-th sampling capacitor is electrically connected to the ground, and another terminal of the M-th sampling capacitor is electrically connected to the M-th input terminal of the first multiplexer and the M-th input terminal of the second multiplexer separately and is electrically connected to the M-th contact of the multi-contact electrode through the M-th channel of the analog switch.

Each of M and N is an integer, N ≥ 2, and N ≥ M ≥ 1.

In one or more embodiments, the pre-filter includes a direct current blocking capacitor unit and a resistor capacitance (RC) low-pass filter unit.

The first voltage follower and the second voltage follower are electrically connected to the direct current blocking capacitor unit separately.

The direct current blocking capacitor unit is electrically connected to the RC low-pass filter unit.

The RC low-pass filter unit is electrically connected to the differential amplifier.

In one or more embodiments, the differential amplifier is a fully differential chopper amplifier.

In one or more embodiments, the low-pass filter is a seventh-order Chebyshev filter.

In one or more embodiments, the analog-to-digital converter is a sigma-delta ADC.

In one or more embodiments, the signal acquisition circuit further includes a single-chip microcomputer.

The single-chip microcomputer is electrically connected to the signal sampling module.

The signal sampling module is configured to acquire an electrical signal between any two contact electrodes based on the multiplexer.

The single-chip microcomputer is configured to perform a fast Fourier transform on the electrical signal acquired by the signal sampling module to obtain multiple corresponding LFP amplitude-frequency curves and select an optimal acquisition contact combination according to the criteria of the highest amplitude A in the β frequency band of an LFP signal and the lowest background noise, where the highest amplitude A in the β frequency band of the LFP signal has priority over the lowest background noise.

The present application further provides a contact combination method applied to the preceding signal acquisition circuit. The contact combination method includes the steps described below.

All channels of the analog switch are closed so that the sampling capacitor array is charged through the multi-contact electrode.

After a preset period of time, all the channels of the analog switch are opened, and the multiplexer is cyclically controlled to select any two sampling capacitors so that sampled data between any two sampling capacitors in the sampling capacitor array is sampled and obtained through the signal sampling module.

A fast Fourier transform is performed on the sampled data between any two sampling capacitors separately so that a set of LFP amplitude-frequency curves is obtained.

An element is selected from the set of LFP amplitude-frequency curves according to the criteria of the highest amplitude in the β frequency band of an LFP signal and the lowest background noise so that an optimal LFP amplitude-frequency curve is obtained.

An optimal acquisition contact combination is obtained according to the optimal LFP amplitude-frequency curve.

In one or more embodiments, the highest amplitude in the β frequency band of the LFP signal has priority over the lowest background noise.

In one or more embodiments, the β frequency band of the LFP signal is 13 to 35 Hz.

The present application further provides a stimulator. The stimulator includes an implantable pulse generator, an electrode lead, and the preceding signal acquisition circuit.

The implantable pulse generator is electrically connected to a multi-contact electrode of the signal acquisition circuit through the electrode lead.

The present application further provides an implantable medical system applied to the preceding signal acquisition circuit. The implantable medical system includes a startup module, a sampling module, a processing module, a selection module, and an output module.

The startup module is configured to close all channels of the analog switch to charge the sampling capacitor array through the multi-contact electrode.

The sampling module is configured to open all the channels of the analog switch after a preset period of time and cyclically control the multiplexer to select any two sampling capacitors, so as to sample and obtain sampled data between any two sampling capacitors in the sampling capacitor array through the signal sampling module.

The processing module is configured to perform a fast Fourier transform on the sampled data between any two sampling capacitors separately to obtain a set of LFP amplitude-frequency curves.

The selection module is configured to select an element from the set of LFP amplitude-frequency curves according to the criteria of the highest amplitude in the β frequency band of an LFP signal and the lowest background noise to obtain an optimal LFP amplitude-frequency curve.

The output module is configured to obtain an optimal acquisition contact combination according to the optimal LFP amplitude-frequency curve.

The present application further provides an implantable medical device. The implantable medical device includes a memory and a processor, where the memory is configured to store a computer program which, when executed by the processor, causes the processor to perform the preceding contact combination method.

### BRIEF DESCRIPTION OF DRAWINGS

FIG. 1 is a block diagram of a signal acquisition circuit according to the present application.
FIG. 2 is another block diagram of a signal acquisition circuit according to the present application.
FIG. 3 is a schematic diagram showing a circuit principle of a signal acquisition circuit according to the present application.
FIG. 4 is a graph showing the relationship between a frequency of an LFP signal and a signal amplitude of the LFP signal according to the present application.
FIG. 5 is a flowchart of a contact combination method according to the present application.
FIG. 6 is a structural diagram of a stimulator according to the present application.
FIG. 7 is a structural diagram of an implantable medical system according to the present application.
FIG. 8 is a structural diagram of an implantable medical device according to the present application.

### DETAILED DESCRIPTION

The present application is described below in conjunction with drawings and embodiments. The embodiments are intended to only illustrate the present application but not to limit the scope of the present application.

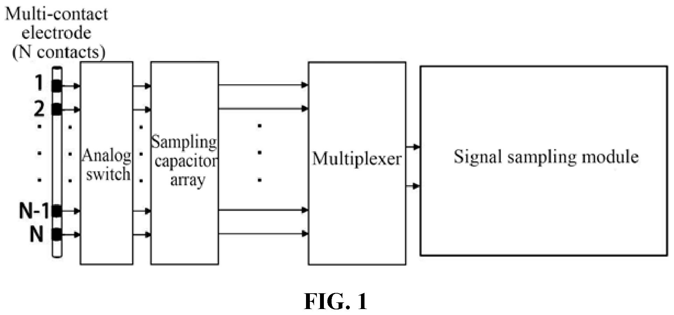

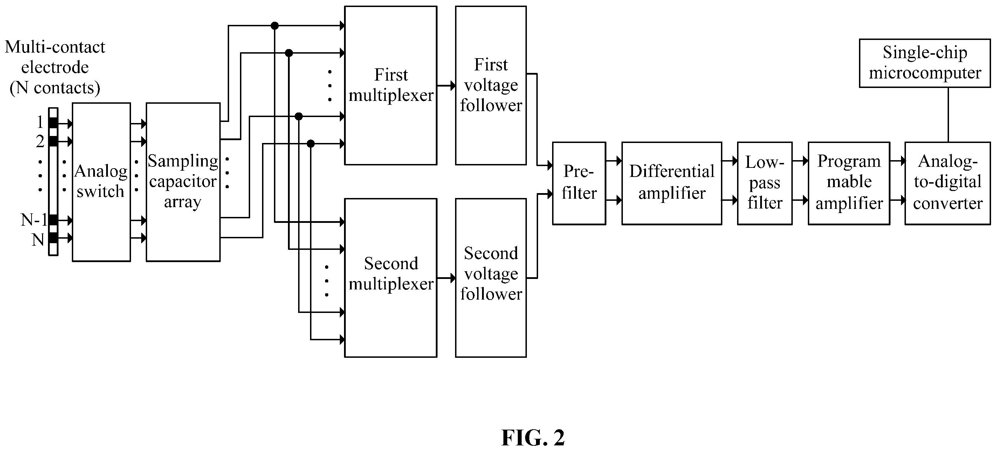

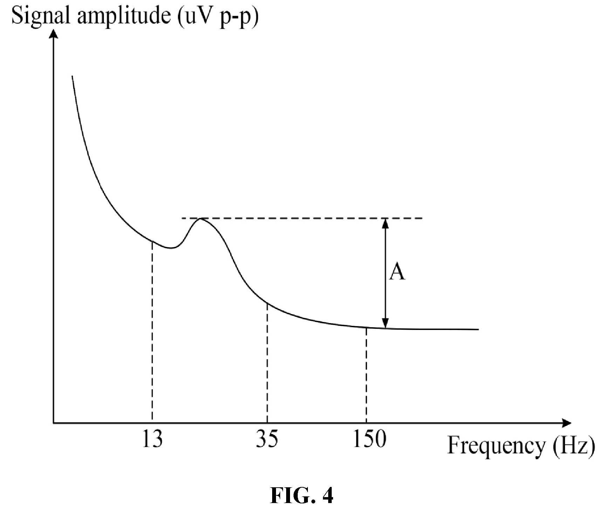

Referring to FIGS. 1 to 4, the present application provides a signal acquisition circuit. The signal acquisition circuit includes a multi-contact electrode, an analog switch, a sampling capacitor array, a multiplexer, and a signal sampling module.

At least part of contacts of the multi-contact electrode are inserted into a target nucleus.

All the contacts of the multi-contact electrode are electrically connected to all sampling capacitors in the sampling capacitor array in a one-to-one correspondence through the analog switch.

Each sampling capacitor in the sampling capacitor array is electrically connected to the multiplexer, so as to enable the multiplexer to select any two sampling capacitors.

The multiplexer is electrically connected to the signal sampling module.

In this embodiment, the signal sampling module is configured to acquire an electrical signal between any two contact electrodes based on the multiplexer. Illustratively, the signal acquisition circuit simultaneously acquires electrical signals of a nucleus in the vicinity of the contacts mainly through the sampling capacitor array and performs cyclical acquisition through the multiplexer, so as to determine an optimal acquisition contact combination. An ADG1414 analog switch may be used as the analog switch. The multiplexer may be one multiplexer in a dual-selection and dual-output mode or may be a combination of two multiplexers each in a single-selection and single-output mode.

The multi-contact electrode may be a nerve stimulation electrode. An electrode lead delivers electrical stimulation to a specific region of a tissue within a body through multiple electrode contacts. The stimulator is provided with one or more electrode leads on one side or two sides. Multiple electrode contacts are disposed on each electrode lead, and the electrode contacts may be uniformly or non-uniformly arranged in the circumferential direction of the electrode lead. As an example, the electrode contacts may be arranged in an array of 4 rows and 3 columns (12 electrode contacts in total) along the circumferential direction of the electrode lead. The electrode contacts may include a stimulation contact and/or an acquisition contact. For example, the electrode contacts may be pad-shaped, ring-shaped, or dot-shaped.

The at least part of the contacts are in use, which may be understood as follows: the multi-contact electrode is in an operating state. That is, the multi-contact electrode is implanted into a particular region within a user's body through an operation or the like. When the multi-contact electrode operates, the particular region is stimulated through the contacts, and/or an electrical signal in the particular region is acquired through the contacts, thereby implementing closed-loop control.

The target nucleus may be a particular region of a specific tissue of a patient, such as a brain tissue, a spinal cord, or limbs. For example, the target nucleus may be the STN, the globus pallidus internal segment (GPi), the ventralis intermedius nucleus (Vim), the anterior limb of the internal capsule (AIC), or the anterior nucleus of thalamus (ANT).

In an embodiment, assuming that the multi-contact electrode has N (N ≥ 2) contacts, the analog switch has N channels, the sampling capacitor array includes N sampling capacitors C₁ to C_{N}, and the multiplexer is constituted by one N-to-2 multiplexer or two N-to-1 multiplexers. During the determination of an optimal acquisition contact combination in the absence of electrical stimulation, the N channels of the analog switch are first simultaneously closed such that each of nuclei coupled to the sampling capacitors charges a respective one of the sampling capacitors C₁ to C_{N} in the sampling capacitor array. After a sufficient period of time, the N channels of the analog switch are simultaneously opened. The multiplexer selects C_{H1} (corresponding to the sampling capacitor C₁) and C_{H2} (corresponding to a sampling capacitor C₂) separately. The signals are processed by a voltage follower, a pre-filter (essentially a band-pass filter), a differential amplifier, a low-pass filter, and a programmable amplifier (that is, a programmable gain amplifier (PGA)) in the signal sampling module and are then sent to an analog-to-digital converter for sampling, thereby obtaining a sampled datum. The multiplexer continues selecting C_{H1} (corresponding to the sampling capacitor C₁), and the multiplexer selects C_{H3} (corresponding to a sampling capacitor C₃) instead of C_{H2}. After subsequent processing, another sampled datum is obtained. The preceding process is cyclically repeated such that N*(N - 1)/2 combinations of sampled data are obtained. A fast Fourier transform (FFT) is performed on the multiple sets of sampled data such that N*(N - 1)/2 LFP amplitude-frequency curves can be obtained. The optimal acquisition contact combination is selected according to the criteria of the highest amplitude A (relative to a background noise baseline beyond 150 Hz) in the β frequency band (13 to 35 Hz) of an LFP signal and the lowest background noise, where the priority of the highest amplitude A in the β frequency band of the LFP signal is higher than the priority of the lowest background noise.

According to the present application, common-mode noise is suppressed through differential LFP sampling, reducing the influence of other physiological signals, such as electrooculogram signals and electrocardiogram signals, on the acquisition of electroencephalogram LFP signals. All the contacts of the multi-contact electrode simultaneously acquire signals, thereby reflecting LFP signals at different positions of the nucleus at the same moment. Subsequently, through combination, pairing, and analysis, the optimal acquisition contact combination for electroencephalogram LFP signal acquisition is identified. Thus, the uncertainty associated with determination results obtained through conventional time-division sampling analysis is eliminated, and the determination accuracy of an optimal acquisition contact can be significantly improved so that an optimal LFP acquisition effect is achieved, thereby implementing real-time optimization of electrical stimulation parameters.

In one or more embodiments, the multiplexer includes a first multiplexer and a second multiplexer.

Each sampling capacitor in the sampling capacitor array is electrically connected to the first multiplexer and the second multiplexer separately, and an output terminal of the first multiplexer and an output terminal of the second multiplexer are each connected to the signal sampling module. Considering the cost and assembly space of the components, the two multiplexers are provided so that a connection between any two sampling capacitors can be rapidly established. Thus, the local field potential between the contacts corresponding to the two sampling capacitors can be acquired.

In one or more embodiments, the signal sampling module includes a first voltage follower, a second voltage follower, the pre-filter, the differential amplifier, the low-pass filter, the programmable amplifier, and the analog-to-digital converter.

The first multiplexer is electrically connected to the first voltage follower.

The second multiplexer is electrically connected to the second voltage follower.

The first voltage follower and the second voltage follower are electrically connected to the pre-filter separately.

The pre-filter is electrically connected to the differential amplifier.

The differential amplifier is electrically connected to the low-pass filter.

The low-pass filter is electrically connected to the programmable amplifier.

The programmable amplifier is electrically connected to the analog-to-digital converter.

In this embodiment, an ADG5206 multiplexer in a single-output mode may be used as each of the first multiplexer and the second multiplexer, and an AD8622 operational amplifier may be used as each of the first voltage follower and the second voltage follower. A positive input terminal of the first voltage follower is electrically connected to the output terminal of the first multiplexer, and a positive input terminal of the second voltage follower is electrically connected to the output terminal of the second multiplexer. A negative input terminal of the first voltage follower is electrically connected to an output terminal of the first voltage follower, and a negative input terminal of the second voltage follower is electrically connected to an output terminal of the second voltage follower. The output terminal of the first voltage follower is electrically connected to an input terminal of the pre-filter, and the output terminal of the second voltage follower is electrically connected to another input terminal of the pre-filter.

The electrical connection in the present application may be a connection implemented through soldering or may be a plug-in connection implemented by providing a plug-in port.

In an embodiment, assuming that the multi-contact electrode has the N (N ≥ 2) contacts, the analog switch has the N channels, the sampling capacitor array includes the N sampling capacitors C₁ to C_{N}, and each of the first multiplexer and the second multiplexer is an N-to-1 multiplexer. During the determination of the optimal acquisition contact combination in the absence of the electrical stimulation, the N channels of the analog switch are first simultaneously closed such that the sampling capacitors C₁ to C_{N} in the sampling capacitor array are charged separately. After the sufficient period of time, the N channels of the analog switch are simultaneously opened. The first multiplexer selects C_{H1} (corresponding to the sampling capacitor C₁), and the signal is buffered by the first voltage follower and sent to one terminal of the pre-filter. The second multiplexer selects C_{H2} (corresponding to the sampling capacitor C₂), and the signal is buffered by the second voltage follower and sent to another terminal of the pre-filter. The signals received by the two terminals are filtered by the pre-filter (essentially the band-pass filter), amplified by the differential amplifier, filtered by the low-pass filter, amplified by the programmable amplifier (that is, the PGA), and sent to the analog-to-digital converter, such as a sigma-delta analog-to-digital converter (ADC), for sampling, thereby obtaining a sampled datum. The first multiplexer continues selecting C_{H1} (corresponding to the sampling capacitor C₁), and the second multiplexer selects C_{H3} (corresponding to the sampling capacitor C₃). After subsequent processing, another sampled datum is obtained. The preceding process is cyclically repeated such that N*(N - 1)/2 combinations of sampled data are obtained. The FFT is performed on the multiple sets of sampled data such that N*(N - 1)/2 LFP amplitude-frequency curves can be obtained. The optimal acquisition contact combination is selected according to the criteria of the highest amplitude A (relative to the background noise baseline beyond 150 Hz) in the β frequency band (13 to 35 Hz) of the LFP signal and the lowest background noise, where the priority of the highest amplitude A in the β frequency band of the LFP signal is higher than the priority of the lowest background noise.

In one or more embodiments, the multi-contact electrode includes the N contacts, the analog switch includes the N channels, the sampling capacitor array includes the N sampling capacitors, and each of the first multiplexer and the second multiplexer is the N-to-1 multiplexer.

One terminal of the M-th sampling capacitor is electrically connected to the ground, and another terminal of the M-th sampling capacitor is electrically connected to the M-th input terminal of the first multiplexer and the M-th input terminal of the second multiplexer separately and is electrically connected to the M-th contact of the multi-contact electrode through the M-th channel of the analog switch.

Each of M and N is an integer, N ≥ 2, and N ≥ M ≥ 1.

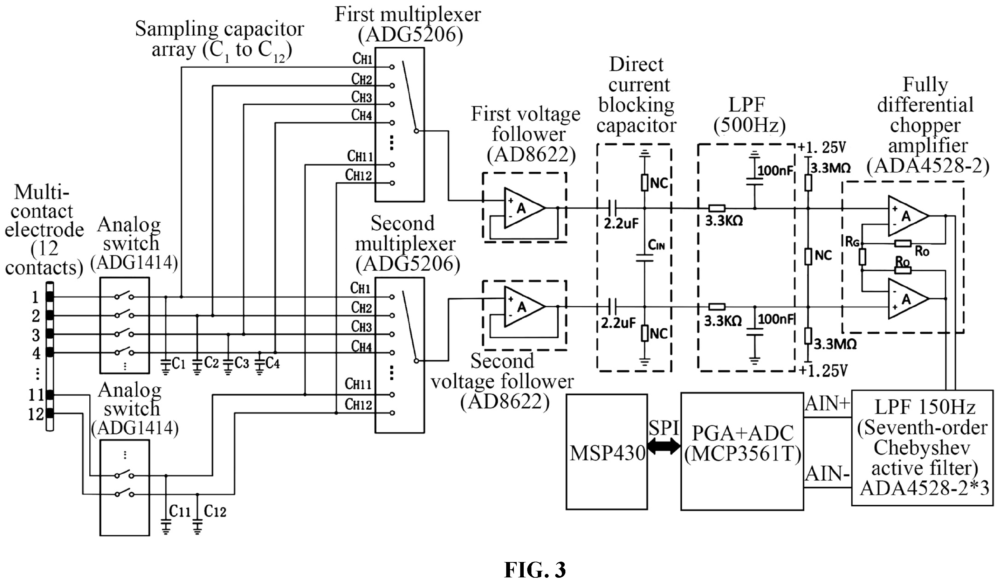

In this embodiment, M represents any number between 1 and N. In an embodiment, referring to FIG. 3, N is set to 12. Then, the multi-contact electrode has 12 contacts, the analog switch has 12 channels (the 12 channels may be provided by two ADG1414 analog switches), the sampling capacitor array includes 12 sampling capacitors C₁ to C₁₂, and each of the first multiplexer and the second multiplexer is a 12-to-1 multiplexer. During the determination of the optimal acquisition contact combination in the absence of the electrical stimulation, the 12 channels of the analog switch are first simultaneously closed such that the sampling capacitors C₁ to C₁₂ in the sampling capacitor array are charged separately. After the sufficient period of time, the 12 channels of the analog switch are simultaneously opened. The first multiplexer selects C_{H1} (corresponding to the sampling capacitor C₁), and the signal is buffered by the first voltage follower and sent to the one terminal of the pre-filter. The second multiplexer selects C_{H2} (corresponding to the sampling capacitor C₂), and the signal is buffered by the second voltage follower and sent to the other terminal of the pre-filter. The signals received by the two terminals are filtered by the pre-filter (essentially the band-pass filter), amplified by the differential amplifier, filtered by the low-pass filter, amplified by the programmable amplifier (that is, the PGA), and sent to the analog-to-digital converter, such as the sigma-delta ADC, for sampling, thereby obtaining a sampled datum. The first multiplexer continues selecting C_{H1} (corresponding to the sampling capacitor C₁), and the second multiplexer selects C_{H3} (corresponding to the sampling capacitor C₃). After subsequent processing, another sampled datum is obtained. The preceding process is cyclically repeated such that 66 combinations of sampled data are obtained. The FFT is performed on the multiple sets of sampled data such that 66 LFP amplitude-frequency curves can be obtained. The optimal acquisition contact combination is selected according to the criteria of the highest amplitude A (relative to the background noise baseline beyond 150 Hz) in the β frequency band (13 to 35 Hz) of the LFP signal and the lowest background noise, where the priority of the highest amplitude A in the β frequency band of the LFP signal is higher the priority of the lowest background noise.

In one or more embodiments, the pre-filter includes a direct current blocking capacitor unit and a resistor capacitance (RC) low-pass filter unit.

The first voltage follower and the second voltage follower are electrically connected to the direct current blocking capacitor unit separately.

The direct current blocking capacitor unit is electrically connected to the RC low-pass filter unit.

The RC low-pass filter unit is electrically connected to the differential amplifier.

In this embodiment, referring to FIG. 3, the direct current blocking capacitor unit may be constituted by two 2.2 µF capacitors, and the RC low-pass filter unit may be constituted by two 3.3 kΩ resistors and two 100 nF capacitors.

In one or more embodiments, the differential amplifier is a fully differential chopper amplifier.

In this embodiment, the fully differential chopper amplifier may be constituted by two ADA4528 operational amplifiers. A signal amplified by the fully differential chopper amplifier may be transmitted to the low-pass filter for subsequent processing.

In one or more embodiments, the low-pass filter is a seventh-order Chebyshev filter.

In this embodiment, the seventh-order Chebyshev filter may be constituted by six ADA4528 operational amplifiers arranged in two groups, where each group includes three operational amplifiers. Data filtered by the seventh-order Chebyshev filter may be transmitted to the programmable amplifier through two interfaces, AIN+ and AIN-.

In one or more embodiments, the analog-to-digital converter is the sigma-delta ADC.

In this embodiment, the sigma-delta ADC may be used as the analog-to-digital converter. During implementation, the analog-to-digital converter integrated with the programmable amplifier, that is, the programmable gain amplifier (PGA), may be used. For example, an MCP3561T data acquisition analog-to-digital converter chip may be used. After analog-to-digital conversion is performed on an analog signal, corresponding sampled data may be outputted through a serial peripheral interface (SPI).

In one or more embodiments, the signal acquisition circuit further includes a single-chip microcomputer.

The single-chip microcomputer is electrically connected to the signal sampling module. Illustratively, the single-chip microcomputer is electrically connected to the analog-to-digital converter. The signal sampling module is configured to acquire an electrical signal between any two contact electrodes based on the multiplexer. The single-chip microcomputer is configured to perform a fast Fourier transform on the electrical signal acquired by the signal sampling module to obtain multiple corresponding LFP amplitude-frequency curves and select an optimal acquisition contact combination according to the criteria of the highest amplitude A in the β frequency band of an LFP signal and the lowest background noise, where the priority of the highest amplitude A in the β frequency band of the LFP signal is higher than the priority of the lowest background noise.

The single-chip microcomputer is configured to perform the fast Fourier transform on the electrical signal acquired by the signal sampling module to obtain the multiple corresponding LFP amplitude-frequency curves and determine, relative to background noise, a contact combination with the highest amplitude of an LFP signal in the β frequency band as the optimal acquisition contact combination.

In this embodiment, the single-chip microcomputer and the analog-to-digital converter may be electrically connected to each other through the SPI data interface. An MSP430 single-chip microcomputer may be used as the single-chip microcomputer. The single-chip microcomputer may perform the FFT on the sampled data provided by the analog-to-digital converter such that the multiple corresponding LFP amplitude-frequency curves are obtained. In addition, the single-chip microcomputer may select the optimal acquisition contact combination according to the criteria of the highest amplitude A(relative to the background noise baseline beyond 150 Hz) in the β frequency band (13 to 35 Hz) of the LFP signal and the lowest background noise, where the priority of the highest amplitude A in the β frequency band of the LFP signal is higher than the priority of the lowest background noise (that is, in a frequency range above 150 Hz).

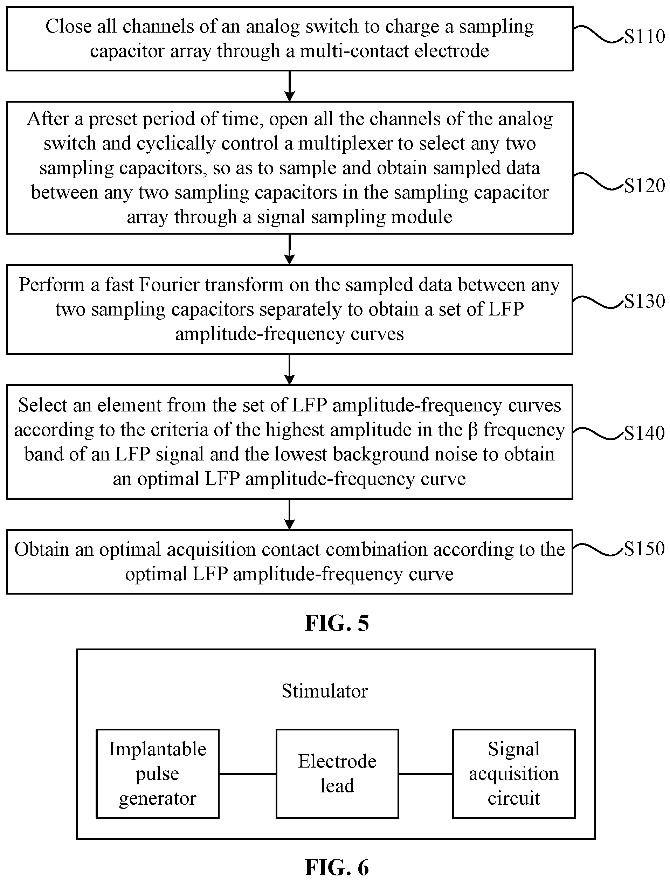

As shown in FIG. 5, the present application further provides a contact combination method applied to the preceding signal acquisition circuit. The contact combination method includes the steps described below.

In S110, all the channels of the analog switch are closed so that the sampling capacitor array is charged through the multi-contact electrode.

In S120, after a preset period of time, all the channels of the analog switch are opened, and the multiplexer is cyclically controlled to select any two sampling capacitors so that sampled data between any two sampling capacitors in the sampling capacitor array is sampled and obtained through the signal sampling module.

In S130, the fast Fourier transform is performed on the sampled data between any two sampling capacitors separately so that a set of LFP amplitude-frequency curves is obtained.

In S140, an element is selected from the set of LFP amplitude-frequency curves according to the criteria of the highest amplitude in the β frequency band of the LFP signal and the lowest background noise so that an optimal LFP amplitude-frequency curve is obtained.

In S150, the optimal acquisition contact combination is obtained according to the optimal LFP amplitude-frequency curve.

In this embodiment, the contact combination method may be understood as a method for determining an optimal acquisition contact combination. The sampling capacitor array is charged through the multi-contact electrode, which may be understood as follows: the multi-contact electrode is used as an acquisition medium to transmit an electrical signal in the nucleus region where each contact is located to a sampling capacitor. Contacts and sampling capacitors are in a one-to-one correspondence. In this manner, targeted charging of the sampling capacitors can be implemented. Electrical energy stored in each sampling capacitor should correspond to a local field potential signal of the nucleus region connected to the sampling capacitor.

The preset period of time may be set to different values according to different types of nuclei or may be set to a fixed value. A specific value of the preset period of time is not limited in the embodiment of the present specification.

Assuming that the multi-contact electrode has N (N ≥ 2) contacts, the analog switch has N channels, the sampling capacitor array includes N sampling capacitors C₁ to C_{N}, and the multiplexer is constituted by one N-to-2 multiplexer or two N-to-1 multiplexers. In one or more embodiments, the multiplexer includes a first multiplexer and a second multiplexer, and the signal sampling module includes a first voltage follower, a second voltage follower, a pre-filter, a differential amplifier, a low-pass filter, a programmable amplifier, and an analog-to-digital converter. During the determination of the optimal acquisition contact combination in the absence of electrical stimulation, the N channels of the analog switch are first simultaneously closed such that the sampling capacitors C₁ to C_{N} in the sampling capacitor array are charged by the N contacts of the multi-contact electrode, respectively. After the preset period of time, the N channels of the analog switch are simultaneously opened. The first multiplexer selects C_{H1} (corresponding to the sampling capacitor C₁), and the signal is buffered by the first voltage follower and sent to one terminal of the pre-filter. The second multiplexer selects C_{H2} (corresponding to a sampling capacitor C₂), and the signal is buffered by the second voltage follower and sent to another terminal of the pre-filter. The signals received by the two terminals are filtered by the pre-filter (essentially a band-pass filter), amplified by the differential amplifier, filtered by the low-pass filter, amplified by the programmable amplifier (that is, a PGA), and sent to the analog-to-digital converter, such as the sigma-delta ADC, for sampling, thereby obtaining a sampled datum. The first multiplexer continues selecting C_{H1} (corresponding to the sampling capacitor C₁), and the second multiplexer selects C_{H3} (corresponding to a sampling capacitor C₃). After subsequent processing, another sampled datum is obtained. The preceding process is cyclically repeated such that N*(N - 1)/2 combinations of sampled data are obtained. In the preceding cyclical process, the channel selection combination of the first multiplexer and the channel selection combination of the second multiplexer cannot be repeated. That is, assuming that a sampled datum has already been obtained by using the combination in which the first multiplexer selects C_{H1} and the second multiplexer selects C_{H3}, the combination in which the first multiplexer selects C_{H1} and the second multiplexer selects C_{H3} cannot be used again, and the combination in which the first multiplexer selects C_{H3} and the second multiplexer selects C_{H1} cannot be used, either. The FFT is performed on different combinations of sampled data such that the set of LFP amplitude-frequency curves constituted by N*(N - 1)/2 LFP amplitude-frequency curves can be obtained. The optimal LFP amplitude-frequency curve representing the optimal acquisition contact combination can be selected according to the criteria of the highest amplitude A (relative to a background noise baseline above 150 Hz) in the β frequency band (13 to 35 Hz) of the LFP signal and the lowest background noise, where the priority of the highest amplitude A baseline beyond in the β frequency band of the LFP signal is higher than the priority of the lowest background noise. The optimal acquisition contact combination can be finally determined through the optimal LFP amplitude-frequency curve.

In one or more embodiments, the highest amplitude in the β frequency band of the LFP signal has the priority over the lowest background noise.

In one or more embodiments, the β frequency band of the LFP signal is 13 to 35 Hz.

As shown in FIG. 6, the present application further provides a stimulator. The stimulator includes an implantable pulse generator, an electrode lead, and the preceding signal acquisition circuit.

The implantable pulse generator is electrically connected to the multi-contact electrode of the signal acquisition circuit through the electrode lead.

In this embodiment, after the optimal acquisition contact combination is determined through the signal acquisition circuit, the implantable pulse generator may apply corresponding electrical signals to the multi-contact electrode in the signal acquisition circuit according to the optimal acquisition contact combination, thereby implementing real-time optimization of electrical stimulation parameters.

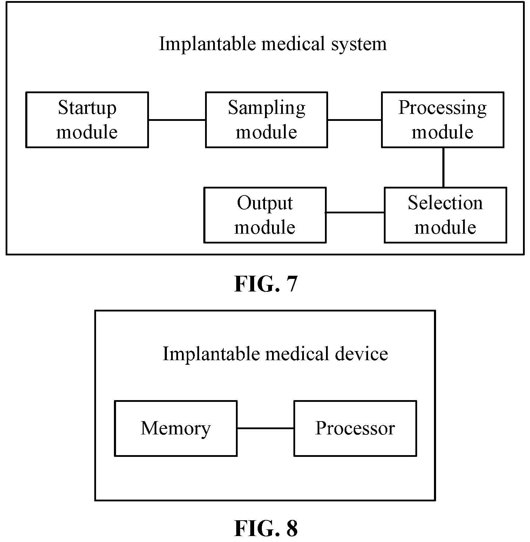

As shown in FIG. 7, the present application further provides an implantable medical system applied to the preceding signal acquisition circuit. The implantable medical system includes a startup module, a sampling module, a processing module, a selection module, and an output module.

The startup module is configured to close all the channels of the analog switch to charge the sampling capacitor array through the multi-contact electrode.

The sampling module is configured to open all the channels of the analog switch after a preset period of time and cyclically control the multiplexer to select any two sampling capacitors, so as to sample and obtain sampled data between any two sampling capacitors in the sampling capacitor array through the signal sampling module.

The processing module is configured to perform the fast Fourier transform on the sampled data between any two sampling capacitors separately to obtain a set of LFP amplitude-frequency curves.

The selection module is configured to select an element from the set of LFP amplitude-frequency curves according to the criteria of the highest amplitude in the β frequency band of an LFP signal and the lowest background noise to obtain an optimal LFP amplitude-frequency curve.

The output module is configured to obtain an optimal acquisition contact combination according to the optimal LFP amplitude-frequency curve.

The implantable medical system may include a stimulator implanted into a patient and a program control device disposed outside the patient. In neuromodulation technology, an electrode is mainly implanted into a specific structure (that is, a target site) in vivo through stereotactic surgery, and an electrical pulse is sent by the stimulator implanted in the patient to the target site via the electrode to modulate electrical activities and functions of a corresponding neural structure and network, thereby reducing symptoms and relieving pains. The stimulator may be any one of an implantable nerve electrical stimulation apparatus, an implantable cardiac electrical stimulation system (also referred to as a cardiac pacemaker), an implantable drug delivery system (IDDS), or a lead switching apparatus. The implantable nerve electrical stimulation apparatus is, for example, a deep brain stimulation (DBS) system, an implantable cortical nerve stimulation (CNS) system, an implantable spinal cord stimulation (SCS) system, an implantable sacral nerve stimulation (SNS) system, or an implantable vagus nerve stimulation (VNS) system. The stimulator may include an IPG, an extension lead, and an electrode lead. The IPG is disposed in the patient in vivo, receives a program control instruction sent by the program control device, provides controllable electrical stimulation energy to a tissue in vivo depending on a sealed battery and circuit, and delivers one or two streams of controllable specific electrical stimulation to a specific region of the tissue in vivo via the implanted extension lead and electrode lead.

The present application provides the signal acquisition circuit, the contact combination method, the stimulator, and the implantable medical system, which can be configured to identify an optimal electrophysiological signal acquisition position of the patient's brain nucleus to achieve the optimal LFP acquisition effect, thereby implementing the real-time optimization of the electrical stimulation parameters.

As shown in FIG. 8, the present application further provides an implantable medical device. The implantable medical device includes a memory and a processor, where the memory is configured to store a computer program which, when executed by the processor, causes the processor to perform the preceding contact combination method.

The preceding embodiments should not limit the present application in any manner, and any technical solutions obtained through equivalent substitutions or equivalent transformations are within the scope of the present application.

## Claims

1. A signal acquisition circuit, comprising a multi-contact electrode, an analog switch, a sampling capacitor array, a multiplexer, and a signal sampling module;
wherein at least part of contacts of the multi-contact electrode are inserted into a target nucleus during use;
all the contacts of the multi-contact electrode are electrically connected to all sampling capacitors in the sampling capacitor array in a one-to-one correspondence through the analog switch;
each sampling capacitor in the sampling capacitor array is electrically connected to the multiplexer, so as to enable the multiplexer to select any two sampling capacitors among all the sampling capacitors; and
the multiplexer is electrically connected to the signal sampling module.

2. The signal acquisition circuit according to claim 1, wherein the multiplexer comprises a first multiplexer and a second multiplexer;
wherein each sampling capacitor in the sampling capacitor array is electrically connected to the first multiplexer and the second multiplexer separately, and an output terminal of the first multiplexer and an output terminal of the second multiplexer are each connected to the signal sampling module.

3. The signal acquisition circuit according to claim 2, wherein the signal sampling module comprises a first voltage follower, a second voltage follower, a pre-filter, a differential amplifier, a low-pass filter, a programmable amplifier, and an analog-to-digital converter;
wherein the first multiplexer is electrically connected to the first voltage follower;
the second multiplexer is electrically connected to the second voltage follower;
the first voltage follower and the second voltage follower are electrically connected to the pre-filter separately;
the pre-filter is electrically connected to the differential amplifier;
the differential amplifier is electrically connected to the low-pass filter;
the low-pass filter is electrically connected to the programmable amplifier; and
the programmable amplifier is electrically connected to the analog-to-digital converter.

4. The signal acquisition circuit according to claim 3, wherein the multi-contact electrode comprises N contacts, the analog switch comprises N channels, the sampling capacitor array comprises N sampling capacitors, and each of the first multiplexer and the second multiplexer is an N-to-1 multiplexer; and
one terminal of an M-th sampling capacitor is electrically connected to the ground, and another terminal of the M-th sampling capacitor is electrically connected to an M-th input terminal of the first multiplexer and an M-th input terminal of the second multiplexer separately and is electrically connected to an M-th contact of the multi-contact electrode through an M-th channel of the analog switch;
wherein each of M and N is an integer, N ≥ 2, and N ≥ M ≥ 1.

5. The signal acquisition circuit according to claim 3, wherein the pre-filter comprises a direct current blocking capacitor unit and a resistor capacitance, RC, low-pass filter unit;
the first voltage follower and the second voltage follower are electrically connected to the direct current blocking capacitor unit separately;
the direct current blocking capacitor unit is electrically connected to the RC low-pass filter unit; and
the RC low-pass filter unit is electrically connected to the differential amplifier.

6. The signal acquisition circuit according to claim 3, wherein the differential amplifier is a fully differential chopper amplifier.

7. The signal acquisition circuit according to claim 3, wherein the low-pass filter is a seventh-order Chebyshev filter.

8. The signal acquisition circuit according to claim 3, wherein the analog-to-digital converter is a sigma-delta analog to digital converter, ADC.

9. The signal acquisition circuit according to claim 1, further comprising a single-chip microcomputer;
the single-chip microcomputer is electrically connected to the signal sampling module.
the signal sampling module is configured to acquire an electrical signal between any two contact electrodes based on the multiplexer; and
the single-chip microcomputer is configured to perform a fast Fourier transform on the electrical signal acquired by the signal sampling module to obtain a plurality of corresponding local field potential, LFP, amplitude-frequency curves and determine, relative to background noise, a contact combination with a highest amplitude of an LFP signal in a β frequency band as an optimal acquisition contact combination.

10. A contact combination method, the method being applied to the signal acquisition circuit according to any one of claims 1 to 9 and comprising:
closing all channels of the analog switch to charge the sampling capacitor array through the multi-contact electrode;
after a preset period of time, opening all the channels of the analog switch and cyclically controlling the multiplexer to select any two sampling capacitors, so as to sample and obtain sampled data between any two sampling capacitors in the sampling capacitor array through the signal sampling module;
performing a fast Fourier transform on the sampled data between any two sampling capacitors separately to obtain a set of local field potential (LFP) amplitude-frequency curves;
selecting an element from the set of LFP amplitude-frequency curves according to criteria of a highest amplitude in a β frequency band of an LFP signal and lowest background noise to obtain an optimal LFP amplitude-frequency curve; and
obtaining an optimal acquisition contact combination according to the optimal LFP amplitude-frequency curve.

11. The contact combination method according to claim 10, wherein a priority of the highest amplitude in the β frequency band of the LFP signal is higher than a priority of the lowest background noise.

12. The contact combination method according to claim 10, wherein the β frequency band of the LFP signal is 13 to 35 Hz.

13. A stimulator, comprising an implantable pulse generator, an electrode lead, and the signal acquisition circuit according to any one of claims 1 to 9;
wherein the implantable pulse generator is electrically connected to the multi-contact electrode in the signal acquisition circuit through the electrode lead.

14. An implantable medical system applied to the signal acquisition circuit according to any one of claims 1 to 9, comprising:
a startup module configured to close all channels of the analog switch to charge the sampling capacitor array through the multi-contact electrode;
a sampling module configured to open all the channels of the analog switch after a preset period of time and cyclically control the multiplexer to select any two sampling capacitors, so as to sample and obtain sampled data between any two sampling capacitors in the sampling capacitor array through the signal sampling module;
a processing module configured to perform a fast Fourier transform on the sampled data between any two sampling capacitors separately to obtain a set of local field potential (LFP) amplitude-frequency curves;
a selection module configured to select an element from the set of LFP amplitude-frequency curves according to criteria of a highest amplitude in a β frequency band of an LFP signal and lowest background noise to obtain an optimal LFP amplitude-frequency curve; and
an output module configured to obtain an optimal acquisition contact combination according to the optimal LFP amplitude-frequency curve.

15. An implantable medical device, comprising a memory and a processor, wherein the memory is configured to store a computer program which, when executed by the processor, causes the processor to perform the contact combination method according to any one of claims 10 to 12.
